# EUROPEAN PATENT APPLICATION

(11) **EP 0 750 916 A2**
(43) Date of publication of application: **02.01.1997**
(21) Application number: 96304195.9
(22) Date of filing: 06.06.1996
(51) Int. Cl.: A61M 25/06, A61M 5/32

(54) **Protective needle cover containment**

(30) Priority: 07.06.1995 US 482593
(71) Applicant: JOHNSON & JOHNSON MEDICAL, INC., Arlington, Texas 76004-3130 (US)
(72) Inventor: van Heugten, Anthony Y., Sarasota, Florida 34243 (US)
(74) Representative: Mercer, Christopher Paul

(57) **Abstract**

An intravenous catheter with a self-locating needle guard. The intravenous catheter comprises a catheter assembly including a catheter attached to a hollow catheter hub, and an introducer needle assembly. The introducer needle assembly includes a hollow needle with a distal tip, with the needle being affixed near its proximal end to a needle hub, the distal end of which is suitable for engagement with the hollow catheter hub. The needle has a substantially round diameter outer shape extending along the distal end thereof, and a nonround deformed outer shape at a given distance from the distal end thereof for engaging a needle guard. The needle guard includes a distal portion having a longitudinal length greater than the given distance and a proximal portion having the same nonround deformed outer shape as the needle to allow the nonround deformed portion of the needle to pass therethrough, but to engage and prevent the round distal end of the needle from passing therethrough. The needle guard is mounted for relative movement with respect to said needle as the needle is withdrawn from the catheter until the nonround deformed shape of the needle guard engages the round distal portion of the needle, at which position the distal portion of the needle guard extends over and guards the needle tip. The nonround deformed outer shape of the needle guard also rotationally orients the nonround deformed portion of the needle, and the needle guard further includes a latch mechanism to engage the lumen of the needle to prevent the needle guard from travelling back toward the proximal end of the needle when the latch mechanism is engaged.

## Description

### BACKGROUND OF THE INVENTION

### 1. Field of the Invention

The present invention relates generally to a protective intravenous (IV) needle cover containment, and more particularly pertains to a protective IV needle cover containment which covers the needle tip after usage to prevent accidental injury from used needles.

### 2. Discussion of the Prior Art

The present invention relates to clinical apparatus of the type in which pointed needles are used to puncture the skin of a patient, and in particular to catheters employing such needles to effectuate venous punctures.

It is well known and common practice by physicians to inject fluids and drugs directly into the bloodstream of patients. Also, during surgical operations, it is frequently necessary to administer whole blood transfusions and parenteral fluids. Historically, introduction of such fluids into the cardiovascular system of a patient has required the making of a venipuncture using a hollow rigid needle having a proximal attachment site for fluid connecting the needle to a source of intravenous fluid or the like. This method of administering fluids created some persisting problems in the art. Primarily, the rigidity of the needle within the vein requires that the needle, usually on the arm, be maintained, for reasons of safety, in a fixed position at the general site of the venipuncture throughout the duration of fluid administration or transfusion, which may consume considerable time. Secondly, where it has been necessary to periodically draw blood samples and/or successively administer intravenous fluids, the patients may be required to experience a venipuncture each time, which repeated venipunctures are generally highly traumatic.

More recently it has been the practice to insert a flexible catheter tube into a vein and leave the catheter tube in such a position for purposes such as periodically administering fluids, transfusions and medication, collecting of blood samples, etc. In this way, the trauma, extravasation, infiltration, etc., of repeated venipunctures are avoided and the danger and discomfort of leaving a rigid needle in the body for a prolonged period of time are overcome. To place the distal end of such a flexible catheter tube within a body cavity, such as a vascular cavity, a cannulated or hollow needle is used to make the venipuncture. Thereafter following the venipuncture, the catheter tube, which is telescopically mounted with respect to the needle, is displaced relative to the needle into the vein of the patient. The needle may thereafter be completely removed from the catheter tube and disposed of. Having been in the patient's body, where it may have been exposed to infectious agents, the needle represents an infection hazard to clinical personnel if they should accidentally jab themselves with it after withdrawal.

Intravenous catheters for the infusion of fluids into the peripheral veins of a patient are frequently produced in two general forms: through-the- needle catheters, in which a catheter is threaded through the needle cannula and into the vein of a patient, and over-the-needle catheters, in which the needle and a concentric outer catheter are inserted into the vein, and the needle is then withdrawn from the emplaced catheter.

A typical over-the-needle IV catheter requires the user to remove and then dispose of a contaminated needle after the needle tip and catheter are properly located in the vein of the patient. Once the needle is withdrawn from the catheter, the user's immediate priorities are infusion set connection and site preparation, including the taping of the catheter to the patient. Because of the urgency of these procedures, the needle is normally just dropped conveniently nearby and then retrieved later. Since the needle at this time is exposed and located close to where the user is completing work with the catheter, accidental self-inflicted needle injuries are not uncommon.

The possibility that clinical personnel might contract conditions such as AIDS or hepatitis through accidental punctures by used needles has been regarded seriously. Accordingly, a significant body of prior art has been developed for preventing such accidental punctures. Now that the range of conditions to which clinical personnel are exposed as a result of accidental needle punctures includes the condition AIDS, it is even more important to provide a safety mechanism which offers such personnel fail-safe protection, i.e. a device which operates without the need for conscious forethought on their part, a mechanism which automatically protects the pointed end of a needle from the moment when it is withdrawn from the body of a patient.

Lemieux U.S. Patent 4,952,207 addresses this problem, and discloses an intravenous catheter which protects a clinician from accidental puncture which may result in the transfer of dangerous infections. The catheter is introduced with the aid of a needle, which is thereafter withdrawn from the patient's body into a protective needle guard housing without exposing the needle tip during the process. The needle guard housing is latched in place after needle withdrawal, and withdrawal and locking are effected in one continuous motion.

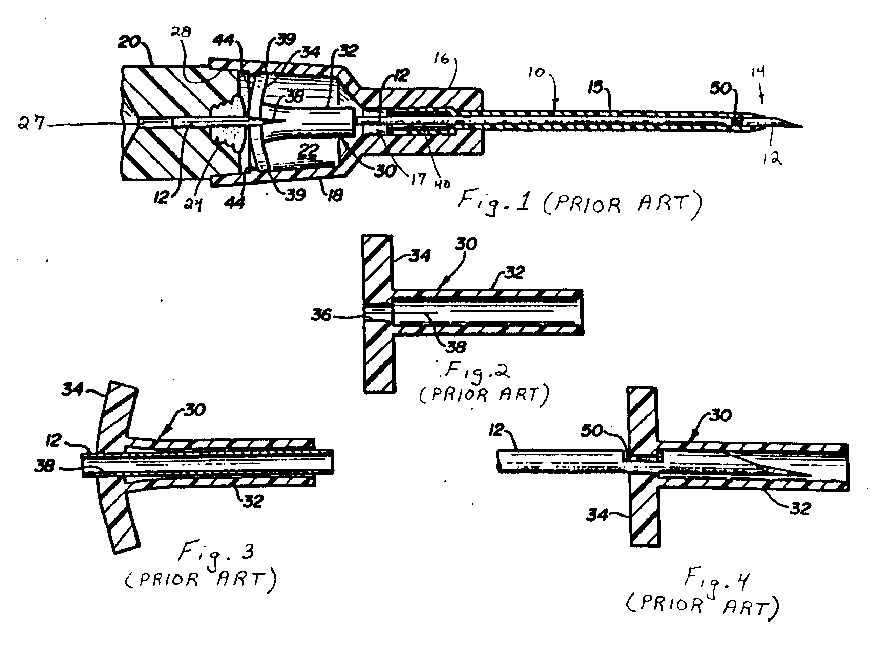

The present invention represents an improvement on the Lemieux patent, which is explained in greater detail with reference to Figures 1-4 herein.

### SUMMARY OF THE INVENTION

Accordingly, it is a primary object of the present invention to provide a protective IV needle cover containment.

A further object of the subject invention is the provision of a protective IV needle cover containment which assures that the needle is secured against accidental puncture after it is withdrawn from the patient's body. It would be desirable for a needle to be securely protected by a small needle guard, and it would be most preferable for the needle guard to be moved into position over the needle tip automatically upon withdrawal of the needle from the patient, without the intervention of any special motion by the user.

In accordance with the teachings herein, the present invention provides an intravenous catheter with a self-locating needle guard. The intravenous catheter comprises a catheter assembly including a catheter attached to a hollow catheter hub, and an introducer needle assembly. The introducer needle assembly includes a hollow needle with a distal tip, with the needle being affixed near its proximal end to a needle hub, the distal end of which is suitable for engagement with the hollow catheter hub. The needle has a substantially round diameter outer shape extending along the distal end thereof, and a nonround deformed outer shape at a given distance from the distal end for engaging a needle guard. The needle guard includes a distal portion having a longitudinal length greater than the given distance and a proximal portion having the same nonround deformed outer shape as the needle to allow the nonround deformed portion of the needle to pass therethrough, but to engage and prevent the round distal end of the needle from passing therethrough. The needle guard is mounted for relative movement with respect to the needle as the needle is withdrawn from the catheter until the nonround deformed shape of the needle guard engages the round distal portion of the needle, at which position the distal portion of the needle guard extends over and guards the needle tip.

In greater detail, the nonround deformed outer shape of the needle guard also rotationally orients the needle, and the needle guard further includes a latch mechanism to engage the lumen of the needle to prevent the needle guard from travelling back toward the proximal end of the needle when the latch mechanism is engaged. The nonround deformed shape can have an oval shape, or a deformed oval shape, or any suitable nonround shape. The distal portion of the needle guard preferably comprises a cylindrical portion having an inner diameter slightly larger than the outer round diameter of said needle.

### BRIEF DESCRIPTION OF THE DRAWINGS

The foregoing objects and advantages of the present invention for a protective needle containment cover may be more readily understood by one skilled in the art with reference being had to the following detailed description of several preferred embodiments thereof, taken in conjunction with the accompanying drawings wherein like elements are designated by identical reference numerals throughout the several views, and in which:
Figure 1 illustrates a cross-sectional view of a prior art IV catheter constructed with a needle guard in place in the catheter hub;
Figure 2 illustrates the needle guard of Figure 1 in cross-sectional detail;
Figure 3 illustrates the needle guard when positioned at the proximal end of the needle;
Figure 4 illustrates the needle guard when locked at the distal end of the needle;
Figure 5 illustrates a cross-sectional view a needle guard in place in a catheter hub constructed pursuant to the teachings of the present invention;
Figure 6 illustrates the needle guard of Figure 5 when positioned at the proximal end of the needle;
Figure 7 illustrates the needle guard of Figure 5 when locked at the distal end of the needle; and
Figures 8 and 9 are sectional views along line A - A in Figure 6 showing cross sectional views of different embodiments of the needle shaft.

### DETAILED DESCRIPTION OF THE DRAWINGS

Referring to the drawings in detail, Figure 1 illustrates a construction for an IV catheter constructed in accordance with the principles of Lemieux U.S. Patent 4,952,207. The catheter 10 comprises a tube 15 made of fluorinated ethylene propylene or polyurethane material. The tube 15 is tapered at its distal end 14 where it may easily slide into an opening in the patient's body formed by a hollow needle 12. The proximal end of catheter tube 15 is concentrically affixed within the distal end of a dual diameter catheter hub 16, 18. The dual diameter hub terminates at its proximal end in a tab or fitting, suitable for attaching the catheter hub to a tubing set which administers a source of intravenous fluid. The catheter 10 is engaged by the hollow needle 12, which is affixed at its proximal end to a needle hub 20.

The proximal end of the catheter 15 is secured within passageway 17 of the distal end 16 of the catheter hub by a sleeve 40. The sleeve 40 is positioned within the proximal end of the lumen of the catheter 15. By forcing sleeve 40 into the lumen of the catheter, the sleeve effects an expansion of the proximal end of the catheter into contact with the internal surface of the passageway 17, thereby locking the hub and catheter together to prevent axial movement therebetween.

The hollow needle 12 extends from its distal tip through the catheter 15 and sleeve 40, and into a passageway 27 in the center of the needle hub 20. The proximal portion of the needle 12 is affixed as by epoxy 24 to the distal end of the needle hub 20. The proximal end of the needle 12 extends beyond the epoxy and into the passageway so that the lumen of the needle is not filled with epoxy during the attachment procedure.

The distal end 28 of the needle hub 20 is of a lesser diameter than the major portion of the needle hub. This lesser diameter end is dimensioned to engage the open proximal end of the catheter hub 18.

A needle point guard 30 is located within a cavity 22 in the catheter hub. Figure 2 is an enlarged cross-sectional view of the needle point guard 30, which resembles a flanged cylinder. The cylindrical distal portion 32 of the guard has an internal aperture with a diameter which enables the portion 32 to slide smoothly along the needle 12. The inner diameter of the cylindrical portion 32 is just slightly larger than the outer diameter of the needle. At the proximal end of the guard 30 is a circular locking flange 34 with a central aperture 36 which is concentric with that of the cylindrical portion 32. The diameter of the flange aperture 36 is slightly less than the outer diameter of the needle 12. The flange 34 and a proximal portion of the cylindrical portion 32 is slit horizontally by an expansion slot 38. The needle point guard 30 is made of a flexible material so that the flange and cylindrical portion will flex open and closed again as described below.

Referring concurrently to Figures 1 and 3, when the catheter and introducer needle are assembled prior to use, the needle point guard is slipped over the point of the needle to slide to a position adjacent the distal end of the needle hub 20. Since the flange aperture 36 is slightly smaller than the outside diameter of the needle, the needle will spread the needle point guard open at the expansion slot 38. The catheter and catheter hub are slipped over the needle until the hub 18 engages the distal end of the needle hub as shown in Figure 1. The needle point guard 30 is thereby enclosed within the cavity 22 of the catheter hub. The spreading of the flange 34 causes the top and bottom of the flange to engage the inner wall of the catheter hub, as indicated at 39. The needle point guard is thus snugly retained within the catheter hub cavity 22. To ensure retention of the needle point guard within the cavity 22, it may further be desirable to form projections extending from the inner wall of the catheter hub just proximal the intended location of the guard, as indicated at 44.

After the user has properly positioned the needle tip and distal end of the catheter within the artery or vein of a patient, as indicated by the presence of blood in a flash changer, not shown, the needle and needle hub are withdrawn from the catheter in preparation for attachment of a tubing set to the catheter hub. As the needle is initially withdrawn, the needle point guard 30 slides along the needle, but remains in position in the cavity 22 due to the engagement of the flange 34 and catheter hub at 39. The needle point guard 30 slides along the needle as shown in Figure 3. Finally, the needle is withdrawn so that an engagement slot 50 near the tip of the needle 12 becomes aligned with the flange 34. At this time, the expansion slot 38 of the guard springs closed as the rim of the flange about aperture 36 is captured in engagement with the slot 50. Since the flange is no longer spread it is no longer retained with the catheter hub, but will leave the cavity 22 in engagement with the needle 12 and slot 50, as shown in Figure 4. The needle may then be safely set aside without danger of accidental injury.

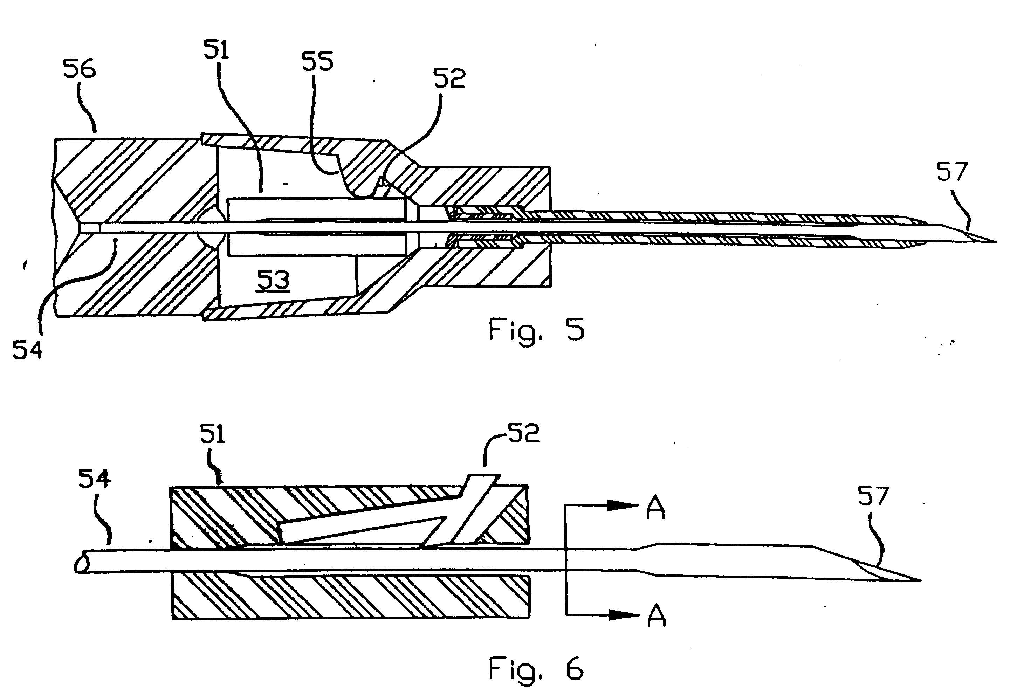

Figure 5 illustrates a sectional view of a needle point guard 51 is located within cavity 53 in the catheter hub 50. Figure 6 is an enlarged cross sectional view of the needle point guard 51 mounted on the proximal end of needle shaft 54, with latch 52 held in an up position by needle shaft 54. In Figure 5, latch 52 is engaged against projection 55, which prevents needle point guard 51 from retracting out of cavity 53 so long as latch 52 is held in the up position by needle shaft 54.

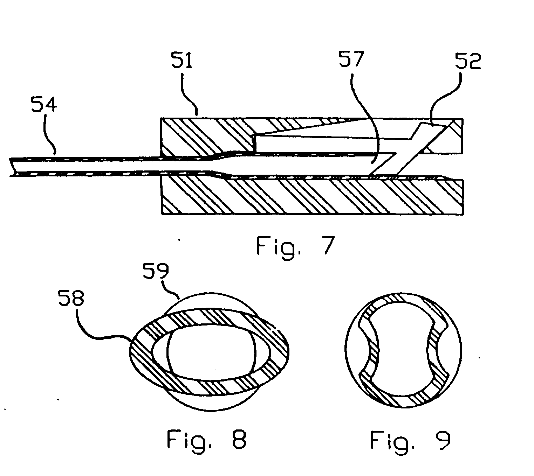

Needle point guard 51 has an aperture shape at its proximal end which conforms in shape to the deformed section of needle shaft 54, shown in one embodiment in Figure 8, deformed section 58, allowing the deformed section 58 to slide freely through the aperture, but not the round, undeformed section 59 of the needle. Needle point guard 51 has an aperture shape at its distal end, of a length greater than the undeformed section of needle shaft 54, and of a shape and size to allow both the deformed and undeformed section of the needle shaft to slide freely thereinto. It is this relationship of aperture shape and needle shaft shape which, as will be described later in further detail, prevents the needle point guard from sliding off and exposing the distal end of the needle shaft.

After the user has properly positioned the needle tip and distal end of the catheter within the artery or vein of a patient, the needle 54 and needle hub 56 are withdrawn from the catheter. As the needle is withdrawn, the needle point guard 51 is retained inside cavity 53 by latch 52. The deformed section of needle 54 slides freely through the needle point guard 51 until the round distal section of needle 54 enters and seats into needle point guard 51, which because of the internal shape of needle guard 51, the round section of needle 54 cannot pass through, preventing needle point guard 51 from sliding off the distal end of needle shaft 54. As the round section of needle 54 seats into needle point guard 51, latch 52 is now directly over the opening of needle lumen 57, and is now no longer being held up by the surface of needle shaft 54, and is driven down into the opening of needle lumen 57 by the spring pressure of latch 52. When driven down, two things occur, first, latch 52 will prevent needle point guard 51 from retracting back on the needle shaft to re-expose the needle point, and second, latch 52 is now clear of projection 55, allowing needle point guard 51 and latch 52 to retract out of cavity 53 while secured onto the distal end of needle shaft 54 as it is removed from the catheter hub.

In Figure 7, latch 52 is shown driven into the opening of needle lumen 57, while needle point guard 51 is at the distal end of the needle shaft 54.

Figures 8 and 9 are sectional views along line A-A in Figure 6 showing cross sectional views of different embodiments of the needle shaft. In the first embodiment of Figure 8, the needle shaft defines an oval deformed shape 58 and a round shape 59. In the second embodiment of Figure 8, the needle shaft defines an inwardly deformed shape 58' and a round shape 59'.

In summary, a portion of the round needle shaft is deformed into an oval shape (or other deformity), and the internal dimensions of the needle guard conform to that shape and size, which allows the protective guard to slide along the deformed oval section but not the round section. This design not only restrains the guard from travelling beyond the distal end of the needle, but also rotationally orients the guard, allowing a latch mechanism to engage the lumen of the needle to prevent the guard from travelling back toward the proximal end of the needle.

While several embodiments and variations of the present invention for a protective needle cover containment are described in detail herein, it should be apparent that the disclosure and teachings of the present invention will suggest many alternative designs to those skilled in the art.

## Claims

1. An intravenous catheter with a self-locating needle guard comprising:
a. a catheter assembly including a catheter attached to a hollow catheter hub;
b. an introducer needle assembly, including a hollow needle with a distal tip, said needle being affixed near its proximal end to a needle hub, the distal end of said needle hub being suitable for engagement with said hollow catheter hub, said needle having a substantially round diameter outer shape extending along the distal end thereof, and a nonround deformed outer shape at a given distance from the distal end thereof for engaging a needle guard; and
c. a needle guard including a distal portion having a longitudinal length greater than said given distance and a proximal portion having said same nonround deformed outer shape as the needle to allow the nonround deformed portion of the needle to pass therethrough, but to engage and prevent the round distal end of the needle from passing therethrough, said needle guard being mounted for relative movement with respect to said needle as said needle is withdrawn from said catheter until said nonround deformed shape of said needle guard engages said round distal portion of said needle, at which position said distal portion of the needle guard extends over and guards said needle tip.

2. An intravenous catheter as claimed in claim 1, wherein the nonround deformed outer shape of the needle guard rotationally orients the nonround deformed portion of the needle, and the needle guard further includes a latch mechanism to engage the lumen of the needle to prevent the needle guard from travelling back toward the proximal end of the needle when the latch mechanism is engaged.

3. An intravenous catheter as claimed in claim 2, wherein the latch includes a projection which locks the needle guard in the hollow catheter hub while the round diameter needle shaft is present and extending through the needle guard.

4. An intravenous catheter as claimed in claim 1, wherein the nonround deformed shape has an oval shape.

5. An intravenous catheter as claimed in claim 1, wherein the nonround deformed shape has an inwardly deformed shape.

6. An intravenous catheter as claimed in claim 1, wherein said distal portion of said needle guard comprises a cylindrical portion having an inner diameter slightly larger than the outer round diameter of said needle.
